# EUROPEAN PATENT APPLICATION

(11) **EP 2 397 472 A1**
(43) Date of publication of application: **21.12.2011**
(21) Application number: 11156286.4
(22) Date of filing: 04.09.2006
(51) Int. Cl.: C07D 249/08, C07C 255/33

(54) **A process for the preparation of a benzylbromide intermediates**

(30) Priority: 30.09.2005 IN MU12162005
(62) Divisional of application: 06842738.4
(71) Applicant: Cadila Healthcare Limited, Ahmedabad 380 015 Gujarat (IN)
(72) Inventor: Solanki, Kirtipalsinh, Saijansinh, 380015, Ahmedabad (IN); Pal, Gautam, 380015, Ahmedabad (IN); Haider, Hussain, 380015, Ahmedabad (IN); Singh, Manoj Kumar, 380015, Ahmedabad (IN); Kothari, Jay Shantilal, 380015, Ahmedabad (IN); Agarwal, Virendra Kumar, 380015, Ahmedabad (IN)
(74) Representative: Clarke, Lionel Paul

(57) **Abstract**

The present invention relates to the improved process for the preparation of 3,5-bis-(1-cyano-1-methylethyl)benzylbromide free from impurities such as 2,2-(5-methyl-1,3-phenylene)-bis(2-methylpropionitrile) and 3,5-bis(1-cyano-1-methylethyl)-α,α-diibromotoluene.

## Description

### FIELD OF INVENTION

The present invention relates to the process for the preparation of pure Anastrozole.

### BACKGROUND OF INVENTION

Aromatase is an enzyme, which effects aromatisation of ring A in the metabolic formation of various steroid hormones. Various cancers for example breast cancer are dependent upon circulating steroid hormones, which have an aromatic ring A. Such cancers can be treated by removing the source of ring A aromatised steroid hormones, for example by the combination of oophorectomy and adrenalectomy. An alternative way of obtaining the same effect is by administering a chemical compound, which inhibits the aromatisation of the steroid ring A.

Anastrozole is a non-steroidal antineoplastic, claimed to inhibit the aromatase (oestrogen synthase) activity. It is useful in the treatment of advanced breast cancer in postmenopausal women.

### PRIOR ART

Synthesis of Anastrozole is reported in Patent No. US 4,935,437 and European Patent Application No. EP 0,296,749. In the above patents the synthesis of Anastrozole is started from intermediate 2,2-(5-methyl-1,3-phenylene)-bis(2-methylpropionitrile) (1), which undergoes bromination to provide 3,5-bis-(1-cyano-1-methylethyl)benzylbromide **(2)** followed by condensation with 1,2,4-triazole **(4)** to form Anastrozole **(5).** (Scheme - 1)

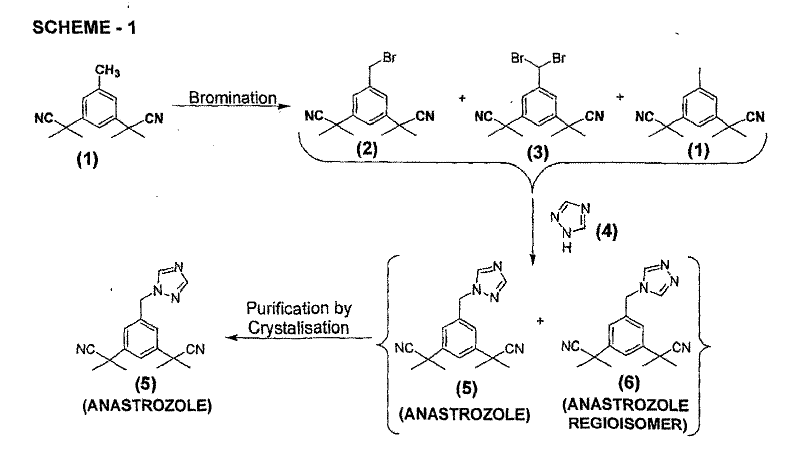

The major problem with the synthesis of Anastrozole is the formation of regioisomer impurity in the said process. The regioisomer **(6),** which forms in 10-20% during the reaction of 3,5-bis-(1-cyano-1-methylethyl)benzylbromide **(2)** with 1,2,4-triazole **(4),** is removed by the purification by crystallization from suitable solvents with a major loss in yield of desired product.

To overcome the formation of regioisomer **(6),** another synthetic route is reported in US Patent No. 4,935,437; in which intermediate **(2)** is reacted with 4-amino-1,2,4-triazole **(7)** to form quaternary ammonium salt **(8),** which further undergoes diazotisation reaction to give Anastrozole **(5)** free from any isomeric impurity (Scheme - 2). This process avoids the formation of Anastrozole regioisomer **(6),** but possibilities of other impurities remain same, which arise from impure intermediate **(2)** contaminated with unreacted starting material **(1)** and dibrominated by-product 3,5-bis(1-cyano-1-methylethyl)-α,α,-dibromotoluene **(3).** Thus the main objective of the present invention relates to an improved process for the preparation of pure Anastrozole **(5)** free from impurities arises due to impure intermediate **(2)** contaminated with unreacted starting material **(1)** and dibrominated by-product 3,5-bis(1-cyano-1-methylethyt)-α,α-dibromotoluene **(3).**

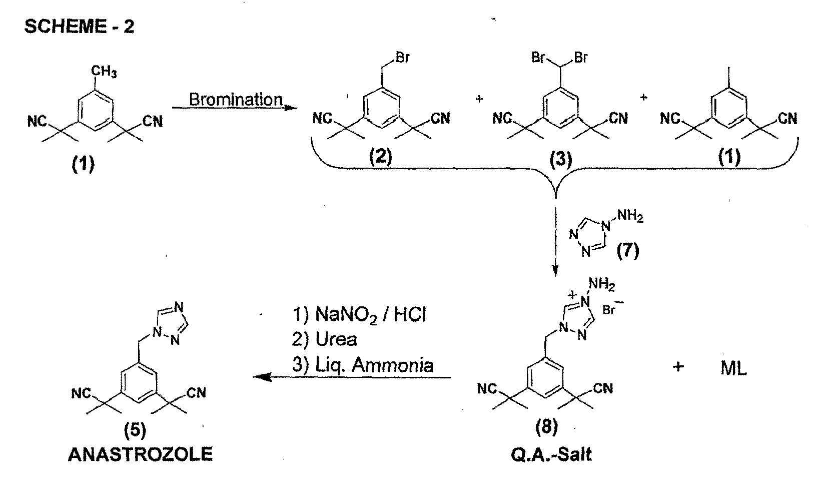

### SUMMARY OF INVENTION

To avoid the formation of regioisomer **(6)** during the formation of Anastrozole **(5);** intermediate **(2)** is treated with 4-amino-1,2,4-triazole **(7)** to form quaternary ammonium salt **(8),** which further undergoes diazotisation reaction to give Anastrozole **(5)** free from any isomeric impurity.

The said process avoids the formation of Anastrozole regioisomer **(6),** but possibilities of other impurities still remain unaffected, which arise from impure intermediate **(2)** contaminated with unreacted starting material **(1)** and dibrominated by-product 3,5 -bis(1-cyano-1-methylethyl)-α,α-dibromotoluene **(3).**

### DETAILED DISCRIPTION OF INVENTION

The present invention relates to an improved process for the preparation of pure Anastrozole **(5)** free from impurities arising due to impure intermediate **(2)** contaminated with unreacted starting material **(1)** and dibrominated by-product 3,5-bis(1-cyano-1-methylethyl)-α,α-dibromotoluene **(3).**

Intermediate **(2)** of more than 80 % HPLC purity contaminated with dibrominated by-product 3,5-bis(1-cyano-1-methylethyl)-α,α-dibromotoluene **(3)** less than 5% and unreacted starting material 2,2-(5-methyl-1,3-phenylene)-bis(2-methylpropionitrile) **(1)** less than 15 %, undergoes condensation with 4-amino-1,2,4-triazole (7) in a suitable solvent to give 4-Amino-1-[3,5-bis-(1-cyano-1-methylethyl)benzyl]-1H-[1,2,4]triazolium bromide (Q.A.-salt) **(8)** in good yield. Quaternary ammonium salt (Q.A.-salt) **(8)** on HPLC analysis shows excellent purity, but still contaminated with 2,2-(5-methyl-1,3-phenylene)-bis(2-methyl-propionitrile) (1) and unreacted 3,5-bis-(1-cyano-1-methylethyl)benzylbromide **(2).** However, HPLC analysis of mother liquor shows that majority of 2,2-(5-methyl-1,3-phenylene)-bis(2-methylpropionitrile) **(1)** and dibrominated by-product 3,5-bis(1-cyano-1-methylethyl)-α,α-dibromotoluene **(3)** are being removed in mother liquor. These results reveal the separations of major part of above impurities in mother liquor during the filtration of Q.A.-salt, but still some extent of related impurities are observed in the HPLC analysis of Q.A.-salt.

Further the Q.A.-salt undergoes diazotisation reaction to give Anastrozole. During the said process, an acidic salt of Anastrozole is prepared in between the process and other impurities are removed by washing acidic Anastrozole salt with an organic solvent. The acidic Anastrozole salt on further basifying and isolation in a suitable solvent affords Anastrozole, which is further purified using organic solvents preferably isopropanol, ethyl acetate or mixture of solvents preferably cyclohexane/ethyl acetate, cyclohexane/isopropanol or a mixture of solvents with water. Thus another embodiment of the present invention relates to the process for the preparation of Anastrozole comprising:
a) reacting brominated product 3,5-bis-(1-cyano-1-methylethyl)benzylbromide of structural formula **(2)** contaminated with 2,2-(5-methyl-1,3-phenylene)-bis(2-methylpropionitrile) of structural formula **(1)** and 3,5-bis(1-cyano-1-methylethyl)-α,α-dibromotoluene of structural formula **(3)** with 4-amino-1,2,4-triazole **(7)** to get quaternary ammonium salt of structural formula **(8),**
b) reacting quaternary salt of structural formula **(8)** with sodium nitrite in presence of an acid,
c) decomposing the excess of nitrous acid,
d) adding an organic solvent to the reaction mixture,
e) basifying the reaction mixture,
f) extracting product in organic layer,
g) adding an acid and water to the solvent layer and extracting product in aqueous layer as acidic salt removing 2,2-(5-methyl-1,3-phenylene)-bis(2-methylpropionitrile) of structural formula **(1)** and 3,5-bis(1-cyano-1-methylethyl)-α,α-dibromotoluene of structural formula **(3),**
h) washing organic layer with water,
i) basifying aqueous layer with a suitable base,
j) extracting product in an organic solvent,
k) isolating Anastrozole which is completely free from 2,2-(5-methyl-1,3-phenylene)-bis(2-methylpropionitrile) of structural formula **(1)** and 3,5-bis(1-cyano-1-methylethyl)-α,α-dibramotoluene of structural formula **(3)** and
l) purifying Anastrozole in a suitable solvent selected from alcohols, ketones, nitriles, esters, hydrocarbons and water either independently or mixture thereof to get all other known / unknown impurities less than 0. 1%.

During the formation of Q.A.-salt **(8),** on reaction of intermediate **(2)** with 4-amino-1,2,4-triazole **(7),** 2,2-(5-methyl-1,3-phenylene)-bis(2-methylpropionitrile) **(1)** and dibrominated by-product **(3)** remain unreacted. HPLC analysis of mother liquor shows the presence of 2,2-(5-methyl-1,3-phenylene)-bis(2-methylpropionitrile) **(1)** and dibrominated by-product **(3)** in mother liquor. However, some extents of these two impurities are also observed in the Q.A.-salt **(8),** which are removed by washing of acidic aqueous layer of Anastrozole with suitable organic solvent.

The present invention also relates to an improved and safe process for the preparation of 3,5-bis-(1-cyano-1-methylethyl)benzylbromide **(2),** which involves replacing hazardous chlorinated solvent with a safe and environmentally more friendly solvents as the reaction media. The preferred solvents for this purpose are selected from hydrocarbons, preferably aliphatic hydrocarbons, more preferably cyclic aliphatic hydrocarbons and most preferably cyclohexane. Thus another embodiment of the present invention relates to an improved process for the preparation of 3,5-bis-(1-cyatio-1-methylethyl)benzylbromide of structural formula **(2)** comprising:
a) reacting 2,2-(5-methyl-1,3-phenylene)-bis(2-methylpropionitrile) of structural formula **(1)** with a brominating reagent in the presence of a catalyst, in the presence of an organic solvent preferably a hydrocarbon and more preferably an aliphatic hydrocarbon,
b) quenching reaction with sodium thiosulphate,
c) removing organic solvent,
d) isolating 3,5-bis-(1-cyano-1-methylethyl)benzylbromide of structural formula **(2)** contaminated with 2,2-(5-methyl-1,3-phenylene)-bis(2-methylpropionitrile) of structural formula **(1)** and 3,5-bis(1-cyano-1-methylethyl)-(α,α-dibromotoluene of structural formula **(3),**
e) crystallising the product from suitable solvents.

Accordingly, the present invention provides aprocess for the preparation of Anastrozole free from 2,2-(5-methyl-1,3-phenylene)-bis(2-methylpropionitrile) of structural formula **(1)** and 3,5-bis(1-cyano-1-methylethyl)-(α,α-dibromotoluene of structural formula **(3)**
each of them preferably less than 0.1% and more preferably below quantitation limits, and all other known / unknown impurities less than 0.1 % comprising:
a) isolating Q.A-salt of structural formula **(8)**
   substantially free from 2,2-(5-methyl-1,3-phenylene)-bis(2-methylpropionitrile) of structural formula (1) and 3,5-bis(1-cyano-1-methylethyl)-α,α-dibromotoluene of structural formula **(3),**
b) removing remaining 2,2-(5-methyl-1,3-phenylene)-bis(2-methylpropionitrile) of structural formula **(1)** and dibromo by-product of structural formula **(3)** from Anastrozole at acidic pH by washing with a suitable solvent,
c) isolating Anastrozole completely free from 2,2-(5-methyl-1,3-phenylene)-bis(2-methylpropionitrile) of structural formula **(1)** and 3,5-bis(1-cyano-1-methylethyl)-α,α-dibromotoluene of structural formula **(3)** at basic pH in the presence of a suitable solvent of the kind such as hereinbefore described and optionally adding an anti-solvent of the kind such as hereinbefore described,
d) purifying Anastrozole in the presence of suitable solvents independently or mixture thereof, optionally using an anti-solvents to get all other known / unknown impurities less than 0.1%.

Preferably, the organic solvent in employed in step (b) is a hydrocarbon, preferably, aromatic hydrocarbon and more preferably toluene.

In another preferred feature, the solvent empployed for extraction in step (c) comprises one or more water immiscible organic solvents.

In another preferred feature, said solvents employed in the purification of Anastrozole in step (d) are selected from alcohols, ketones, nitriles, esters, hydrocarbons and water independently or mixture thereof.

In another embodiment, the present invention provides an improved process for the preparation of 3,5-bis-(1-cyano-1-methylethyl)benzylbromide of structural formula **(2)** comprising:
a) reacting 2,2-(5-methyl-1,3-phenylene)-bis(2-methyl-propionitrile) of structural formula **(1)** with a brominating reagent in the presence of a catalyst, in a suitable solvent preferably a hydrocarbon,
b) quenching reaction with sodium thiosulphate,
c) removing solvent and isolating said 3,5-bis-(1-cyano-1-methylethyl)benzylbromide of structural formula **(2)** contaminated with said 2,2-(5-methyl-1,3-phenylene)-bis(2-methylpropionitrile) of structural formula **(1)** and 3,5-bis(1-cyano-1-methylethyl)-(α,α-dibromotoluene of structural formula **(3),**
   and
d) crystallising the product in the presence of a suitable solvent of the kind such as hereinbefore described.

In another preferred feature, said hydrocarbon employed in step (a) is preferably an aliphatic hydrocarbon and more preferably, a aliphatic cyclic hydrocarbon.

In another preferred feature, the said aliphatic cyclic hydrocarbon is cyclohexane.

In another preferred feature, the said brominating reagent used in operation (a) is preferably N-bromosuccinimide or 1,3-dibromo-5,5-dimethylhydantoin.

In another preferred feature, said solvent employed in said crystallization is an alcohol preferably, a C₁-C₁₀ and more preferably, methanol or isopropanol.

The present invention will now be described with reference to the following non-limiting examples which are merely for the purposes of illustrating the invention. It will be apparent to a person skilled in the art that various modifications may be possible within the present invention without departing from the scope thereof.

### Example -1

### 3,5-bis-(1-cyano-1-methylethyl)benzylbromide (2)

In a 5 L R. B. Flask, fitted with double surface condenser and thermometer pocket on a water bath cyclohexane (1.5 L), 2,2-(5-methyl-1,3-phenylene)-bis(2-methylpropionitrile) (1) (50 g), 2,2-Azo-bis(isobutyronitrile) (AIBN) (2 g) and 1,3-dibromo-5,5-dimethylhydantoin (130 g) were charged at 25 - 35 °C. The reaction mixture was heated at 80 - 85 °C with vigorous stirring for 4 hours. The reaction mass was cooled to 25 - 35 °C and washed with water (500 mL x 2) followed by washing with 2% aqueous solution of sodium thiosulphate (500 mL). Cyclohexane layer was separated and distilled under vacuum at 50-55 °C to get residue, which was crystallised from cyclohexane (250 mL) and further dried at 55 - 60 °C for 3 hours, to get crude product 52 gm, which on crystallization from methanol gave product (37 gm). HPLC-purity shows 3,5-bis-(1-cyano-1-methylethyl)benzyibromide (**2**) as product >80%, contaminated with unreacted 2,2-(5-methyl-,3-phenylene)-bis(2-methylpropionitrile) (**1**) <15 % and dibrominated by-product 3,5-bis(1-cyano-1-methylethyl)-α,α-dibromotoluene (3) < 5%.

### Example - 2

### 4-Amino-1-[3,5-bis-(1-cyano-1-methylethyl)benzyl]-1H-[1,2,4]triazolium bromide (8) (Q. A.-Salt)

Isopropanol (1.5 L), 3,5-bis-(1-cyano-1-methylethyl)benzylbromide **(2)** (100 g) and 4-amino-1,2,4-triazole (22.5 g) were charged in a flask at room temperature. The reaction mass was heated at 80 - 85 °C for next 5 hours. The reaction mass was further cooled down to room temperature and stirred for next one hour at 25 - 35 °C. The precipitated solid QA-salt was filtered and washed with fresh isopropanol (100 mL x 2). The Q.A.-salt was dried at 60 - 70 °C till LOD is less than 1.0 %, giving solid product (74.4 g). m.p.: 198 - 200 °C.

### Example - 3

### 2,2'-[5-(1H-1,2,4-Triazol-1-ylmethyl)-1,3-phenylene]di(2-methylpropiononitrile) (5), Anastrozole

4-Amino-1-[3,5-bis-(1-cyano-1-methylethyl)benzyl]-1H-[1,2,4]triazolium bromide **(8)** (70 g) was dissolved in conc. HCl (280 mL) in a 5 L R.B. flask and cooled to -5 °C. A solution of sodium nitrite (15 g) in water (70 mL) was slowly added to the reaction mixture at 0 - 5 °C in 4 hrs and the reaction mixture was stirred for one hour at 0 - 5 °C and further at 10 - 20 °C for next 3 hours. The reaction mixture was quenched by the addition of a solution of urea (4.5 g) in water (15 mL). Toluene (700 mL) was added to the reaction mixture and the heterogeneous solution was further cooled down to 0 - 5 °C. The solution was basified by the addition of liquor ammonia (365 mL) slowly in 4 hours at 5 - 25°C. Organic layer was separated and further washed with water (200 mL). Aqueous layer was removed and a solution of conc. HCl (140 mL) in water (140 mL) was added to the organic layer slowly in 30 minutes at 25 - 30 °C and reaction mass was heated at 60 - 65 °C for 30, minutes. The lower aqueous layer (280 - 300 mL) containing product was collected in a conical flask maintaining at 50°C. The aqueous part was again washed with toluene (700 mL) at 60 - 65 °C for 30 minutes. The lower aqueous layer, containing product was charged in a separating funnel and again washed with fresh toluene (700 mL). The aqueous layer, containing product was transferred in a R.B. flask and ethyl acetate (350 mL) was added to it. The heterogeneous solution was cooled to 0 - 5 °C basified by the slow addition of liquor ammonia (280 mL) in 2 - 3 hours at 5 - 25 °C. The solution was stirred for one hour at 25 - 35 °C, and the upper organic layer (360 - 375 mL) containing product was separated and filtered through hyflow super cell bed. Solvent was distilled out below 50 °C under vacuum leaving approximately 100 mL ethyl acetate in the flask. The content of the flask was cooled down to 25 - 35 °C and cyclohexane (500 mL) was added to the solution slowly in 30 minutes. The precipitated solid product was filtered and washed with fresh cyclohexane (20 mL x 2). The product was dried at 45 - 50 °C till LOD was less than 1.0 % to get Anastrozole (44 g). HPLC-purity: not less than 98%.

### Example - 4

### Purification of Anastrozole (Ethyl acetate/Cyclohexane)

In a R.B. flask Anastrozole (32 g) from example - 3 was dissolved completely in ethyl acetate (64 mL) at 35 - 40 °C. The solution was cooled down to 25 - 35 °C and cyclohexane (320 mL) was added slowly to the solution under stirring in 1 hour. The solution was further stirred at 25 - 35 °C for 2 hours, and the precipitated solid product was filtered, washed with fresh cyclohexane (15 mL x 2) at 25 - 35 °C. The solid product was dried at 50 °C, till LOD was less than 0.5 %. Weight of dry Anastrozole: 30 g. HPLC-purity : not less than 99.5%.

### Example - 5

### Purification of Anastrozole (Isopropanol / Cyclohexane)

Anastrozole (110 g) from example - 3 was dissolved in isopropanol (330 mL) at 45 - 50 °C. The solution was cooled down to 25 - 35 °C and cyclohexane (330 mL) was added drop wise in 30 minutes. The solution was stirred at 25 - 35 °C for 2 hours; the precipitated solid product was filtered and washed with fresh cyclohexane (30 mL x 2) and dried at 50 °C to get 92 g of pure Anastrozole. HPLC-purity: not less than 99.5%.

### Example - 6

### Purification of Anastrozole (Ethanol / Cyclohexane)

Anastrozole (5 g) from example - 3 was dissolved in ethanol (10 mL) at 45 - 50 °C. The solution was cooled down to 25 - 35 °C and cyclohexane (50 mL) was added drop wise in 30 minutes. The solution was stirred at 25 - 35 °C for 2 hours; the precipitated solid product was filtered and washed with fresh cyclohexane (5 mL x 2) and dried at 50 °C to get 4.0 g of pure Anastrozole. HPLC-purity: not less than 99:5%.

### Example - 7

### Purification of Anastrozole (Isopropanol)

Anastrozole (5 g) from example - 3 was dissolved in isopropanol (20 mL) at 50 - 55 °C. The solution was cooled down to 5 - 10 °C and stirred for 2 hours; the precipitated solid product was filtered and washed with chilled fresh isopropanol (5 mL) and dried at 50 °C to get 4.5 g of pure Anastrozole. HPLC-purity: not less than 99.5%.

### Example - 8

### Purification of Anastrozole (Isopropanol/Water)

Anastrozole (5 g) from example - 3 was dissolved in isopropanol (5 mL) and water (20 mL) at 65 - 70 °C. The solution was cooled down to 25 - 35 °C and stirred for 1 hour; the precipitated solid product was filtered and dried at 50 °C to get 4.5 g of pure Anastrozole. HPLC-purity: not less than 99.5%.

### Example - 9

### Purification of Anastrozole (Ethanol/Water)

Anastrozole (5 g) from example - 3 was dissolved in ethanol (5 mL) and water (40 mL) at 50 - 55 °C. The solution was cooled down to 25 - 35 °C and stirred for 1 hour; the precipitated solid product was filtered and dried at 50 °C to get 4.5 g of pure Anastrozole. HPLC-purity: not less than 99.5%.

### Example -10

### Purification of Anastrozole (Methanol/Water)

Anastrozole (5 g) from example - 3 was dissolved in methanol (5 mL) and water (20 mL) at 50 - 55 °C. The solution was cooled down to 25 - 35 °C stirred for 2 hours; the precipitated solid product was filtered and dried at 50 °C to get 4.5 g of pure Anastrozole. HPLC-purity: not less than 99.5%.

The following constitute particularly preferred numbered embodiments of the present invention:
Numbered Embodiment 1. A process for the preparation of Anastrozole free from 2,2-(5-methyl-1,3- phenylene)-bis(2-methylpropionitrile) of structural formula (1) 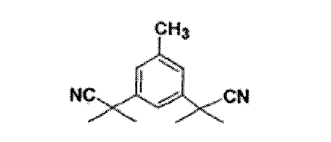 and 3,5-bis(1-cyano-1-methylethyl)-α,α-dibromotoluene of structural formula (3) 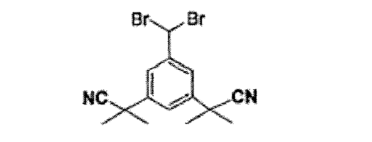 each of them preferably less than 0.1% and more preferably below quantitation limits, and all other known / unknown impurities less than 0.1 % comprising:
   a) isolating Q.A-salt of structural formula (8) 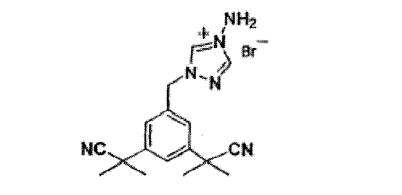 substantially free from 2,2-(5-methyl-1 ,3-phenylene)-bis(2-methylpropionitrile) of structural formula (1) and 3,5-bis(1-cyano-1-methylethyl)-α,α-dibromotoluene of structural formula (3),
   b) removing remaining 2,2-(5-methyl-1,3-phenylene)-bis(2-methylpropio nitrile) of structural formula (1) and dibromo by-product of structural formula (3) from Anastrozole at acidic pH by washing with a suitable solvent,
   c) isolating Anastrozole completely free from 2,2-(5-methyl-1,3-phenylene)- bis(2-methylpropionitrile) of structural formula (1) and 3,5-bis(1-cyano-1-methylethyl)-α,α-dibrotnotoluene of structural formula (3) at basic pH in the presence of a suitable solvent of the kind such as hereinbefore described and optionally adding an anti-solvent of the kind such as hereinbefore described
   d) purifying Anastrozole in the presence of suitable solvents independently or mixture thereof, optionally using an anti-solvents to get all other known / unknown impurities less than 0.1%.
Numbered Embodiment 2. A process Numbered Embodiment 1 wherein the organic solvent in employed in step (b) is a hydrocarbon, preferably, aromatic hydrocarbon and more preferably toluene.
Numbered Embodiment 3. A process according to Numbered Embodiment 1 or 2 wherein the solvent used for extraction in step (c) comprises one or more water immiscible organic solvents.
Numbered Embodiment 4. A process according to any preceding Numbered Embodiment wherein said solvents employed in the purification of Anastrozole in step (d) are selected from alcohols, ketones, nitriles, esters, hydrocarbons and water independently or mixture thereof.
Numbered Embodiment 5. An improved process for the preparation of 3,5-bis-(1-cyano-1-methylethyl)benzylbromide of structural formula (2) comprising: 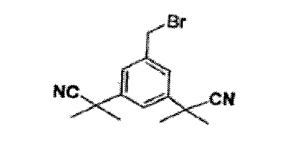
   a) reacting 2,2-(5-methyl-1,3-phenylene)-bis(2-methyl-propionitrile) of structural formula (1) with a brominating reagent in the presence of a catalyst, in a suitable solvent preferably a hydrocarbon,
   b) quenching reaction with sodium thiosulphate,
   c) removing solvent and isolating said 3,5-bis-(1-cyano-1-methylethyl) benzylbromide of structural formula (2) contaminated with said 2,2-(5-methyl-1,3-phenylene)-bis(2-methylpropionitrile) of structural formula (1) and 3,5-bis(1-cyano-1-methylethyl)-α,α-diibromotoluene of structural formula (3), 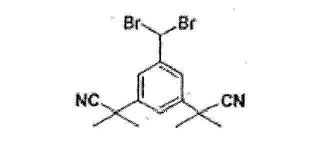 and
   d) crystallising the product in the presence of a suitable solvent of the kind such as hereinbefore described.
Numbered Embodiment 6. A process according to Numbered Embodiment 5 wherein the said hydrocarbon employed in step (a) is an aliphatic hydrocarbon.
Numbered Embodiment 7. A process according to Numbered Embodiment 6 wherein the said aliphatic hydrocarbon is a aliphatic cyclic hydrocarbon.
Numbered Embodiment 8. A process according to Numbered Embodiment 7 wherein the said aliphatic cyclic hydrocarbon is cyclohexane.
Numbered Embodiment 9. A process according to any of Numbered Embodiments 5 to 8 wherein the said brominating reagent employed in step (a) is preferably N-bromosuccinimide or 1,3-dibromo- 5,5-dimethyihydantoin.
Numbered Embodiment 10. A process according to any of Numbered Embodiments 5 to 9 wherein said solvent employed in said crystallization is an alcohol preferably, a C₁-C₁₀ and more preferably, methanol or isopropanol.

## Claims

1. An improved process for the preparation of 3,5-bis-(1-cyano-1-methylethyl)benzylbromide of structural formula (2) comprising: 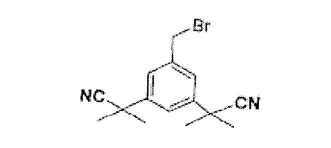
a) reacting 2,2-(5-methyl-1,3-phenylene)-bis(2-methyl-propionitrile) of structural formula (1) 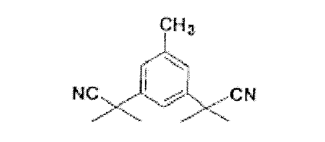 with a brominating reagent in the presence of a catalyst, in a suitable solvent, preferably a hydrocarbon,
b) quenching the reaction with sodium thiosulphate,
c) removing solvent and isolating said 3,5-bis-(1-cyano-1-methylethyl) benzylbromide of structural formula (2) contaminated with said 2,2-(5-methyl-1,3-phenylene)-bis(2-methylpropionitrile) of structural formula (1) and 3,5-bis(1-cyano-1-methylethyl)-α,α-diibromotoluene of structural formula (3), 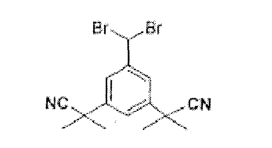 and
d) crystallising the product in the presence of a suitable solvent

2. A process as claimed in claim 1, wherein the solvent in step d) is selected from the group consisting of alcohols, ketones, nitriles, esters, hydrocarbons and water, or a mixture thereof.

3. A process as claimed in claim 1 or claim 2 wherein the said hydrocarbon employed in step (a) is an aliphatic hydrocarbon.

4. A process as claimed in claim 3 wherein the said aliphatic hydrocarbon is a aliphatic cyclic hydrocarbon.

5. A process as claimed in claim 4 wherein the said aliphatic cyclic hydrocarbon is cyclohexane.

6. A process as claimed in any preceding claim wherein the said brominating reagent employed in step (a) is N-bromosuccinimide or 1,3-dibromo-5,5-dimethyihydantoin.

7. A process as claimed in any preceding claim wherein said solvent employed in said crystallization is an alcohol

8. A process as claimed in claim 7, wherein the alcohol is a C₁-C₁₀ alcohol.

9. A process as claimed in claim 8, wherein the alcohol is methanol or isopropanol.
